# EUROPEAN PATENT APPLICATION

(11) **EP 4 835 471 A1**
(43) Date of publication of application: **04.11.2026**
(21) Application number: 25173637.7
(22) Date of filing: 30.04.2025
(51) Int. Cl.: G01N 33/52, G01N 33/543, C12Q 1/00, B05D 1/00, B05D 3/14

(54) **A METHOD FOR FUNCTIONALIZING A SURFACE WITH A BIOMOLECULE IN A LAYER HAVING ANTIFOULING PROPERTIES**

(71) Applicant: Imec VZW, 3001 Leuven (BE); Katholieke Universiteit Leuven, 3000 Leuven (BE); Molecular Plasma Group BV, 3001 Leuven (BE)
(72) Inventor: Dekoster, Tijs, 3020 Herent (BE); Jans, Karolien, 3980 Tessenderlo (BE); Vos, Rita, 3128 Baal (BE); Van Roy, Willem, 3360 Bierbeek (BE); Delabie, Annelies, 3360 Bierbeek (BE)
(74) Representative: Roth, Sebastian

(57) **Abstract**

The present disclosure relates to functionalizing a surface of a biosensor with a biomolecule. The disclosure provides a method for codepositing the biomolecule and an organic precursor onto the surface, and provides a biosensor with a functionalized surface. The method comprises a step of codepositing the biomolecule and an organic precursor onto the surface using atmospheric pressure plasma deposition. To obtain a stable layer with antifouling properties on the surface, the organic precursor comprises a polymerizable group and an ethylene oxide.

## Description

### TECHNICAL FIELD

The present disclosure relates to functionalizing a surface of a biosensor with a biomolecule. To this end, the disclosure provides a method for codepositing the biomolecule and an organic precursor onto the surface. The method leads to the incorporation of the biomolecule into a stable biocompatible polymer layer with antifouling properties.

### BACKGROUND

Biosensors play an increasingly important role in our daily life, with applications going from measuring the glucose level in the blood to detecting infectious diseases. Crucial for most biosensors is the functionalization of a surface with a biomolecule of interest using a linker layer. To deposit the linker layer, new technologies are investigated, such as atmospheric pressure plasma deposition (APPD). This technology potentially allows the biomolecule deposition in a more cost-effective and scalable way.

During APPD, a plasma of a carrier gas, such as helium (He), argon (Ar), or nitrogen (N₂), is generated in between two electrodes, and is blown out of the region in between the electrodes by a flow of the carrier gas. A precursor is injected into the afterglow of the plasma, to limit fragmentation and to allow the deposition of fragile biomolecules and organic precursors.

A first exemplary approach of using APPD to deposit a biomolecule onto a substrate surface, which will provide the surface, comprises a two-step process. In this process, an organic linker layer is deposited in a first step, and the biomolecule is deposited on the linker layer in a second step. A two-step process is, however, not ideal. A second exemplary approach comprises a one-step process, wherein an aerosol of a biomolecule and vapor of a hydrocarbon precursor (such as ethylene or acetylene) are copolymerized by the plasma during APPD onto a surface. However, this approach leads to a carbon rich linker layer with unfavorable properties for both the survival of the biomolecule and the fouling of the surface.

### SUMMARY

In view of the above, this disclosure has the objective to provide a method for functionalizing a surface of a biosensor with a biomolecule, wherein the method overcomes the issues of the conventional approaches that use APPD. For instance, an objective is to enable the functionalization in a one-step APPD process. Another objective is to have antifouling properties, to prevent the layer on the surface from fouling, for example, by proteins or cells. Another objective is to achieve sufficient cross-linking to avoid dissolution of the layer in aqueous solutions and prevent leaching of the biomolecules. Another objective is to retain the bioactivity and stability of the biomolecules.

These and other objectives are achieved by the solutions of this disclosure, which are described in the independent claims. Advantageous implementations are described in the dependent claims.

A first aspect of this disclosure provides a method for functionalizing a surface of a biosensor with a biomolecule, the method comprising: codepositing the biomolecule and an organic precursor onto the surface using APPD; wherein the organic precursor comprises a polymerizable group and an ethylene oxide functional group.

The use of the APPD technology in the method of the first aspect enables co-injection of the biomolecule and the organic precursor, can be performed at atmospheric pressure and with a mild plasma, is solvent free, and is suitable for the treatment of large surface areas.

The method of the first aspects enables a one-step process, and produces a film on the surface, which shows good cross-linking to prevent dissolution in aqueous solutions, has antifouling properties - due to the selection of the organic precursor
- that prevent fouling by proteins or cells, and immobilizes the biomolecules on the senor surface while retaining their bioactivity and stability.

A biomolecule is a chemical compound naturally found in living organisms, examples including proteins, nucleic acids, carbohydrates, and lipids. Notably, the "biomolecule" in this case means a type of biomolecule. That is, the term "biomolecule" may not strictly refer to a single "instance" of the biomolecule, i.e. an individual molecule, but to a class, and many copies of that biomolecule can be deposited.

During the deposition, the surface may strictly speaking be still be a surface of a substrate. The substrate with the functionalized surface may subsequently be used to process the complete biosensor, for example, by processing a transducer of the biosensor. The "surface of a biosensor" in this disclosure also relates to such situations.

A polymerizable group may be a reactive moiety, like vinyl or acrylate, that enables monomers to bond and form polymers through polymerization.

A biosensor is a combination of a functionalized surface including a bio-receptor - e.g. the surface functionalized with the biomolecule - and a transducer. The bio-receptor (or short receptor) has sensitivity and selectivity to a target, e.g., an analyte that may bind to the bio-receptor. A transducer of a biosensor is an electrical or optical component that converts the biochemical interaction of e.g. the target with the bio-receptor into an electronic signal.

In an implementation of the method, the polymerizable group of the organic precursor comprises a vinyl, or an acrylate, or a methacrylate, or an epoxy, or a silane.

In an implementation of the method, the organic precursor further comprises a spacer, the spacer comprising an alkane, or an ether, or a ketone, or an ester, or an amide.

The spacer may be used to increase the anti-fouling properties of the functionalized surface, and the ratio reactive group over length also determines the density and/or porosity of the layer (or film) on the surface, and additionally enables to crosslink the biomolecule into the formed polymer.

In an implementation of the method, the biomolecule is one of an enzyme, an antibody, a nanobody, an aptamer, DNA, RNA, and a peptide.

In an implementation of the method, the organic precursor is di(ethylene glycol) divinyl ether or poly(ethylene glycol) dimethacrylate, and/or the biomolecule is glucose oxidase.

In an implementation of the method, the biomolecule and the organic precursor are codeposited assisted by a plasma afterglow during the APPD or are codeposited and then treated with the plasma afterglow.

In an implementation of the method, during the APPD, the organic precursor is injected as vapor or an aerosol into the plasma afterglow, and the biomolecule is injected as an aerosol into the plasma afterglow.

A suitable solvent for the biomolecule aerosol may be water.

In an implementation of the method, the biomolecule and the organic precursor are dissolved in water, and during the APPD the water comprising the dissolved organic precursor and biomolecule is injected into the plasma afterglow.

For example, the biomolecule and the organic precursor may be dissolved in water e.g., 25 mg/ml glucose oxidase in water, containing also 10V% poly(ethylene glycol) dimethacrylate.

In an implementation of the method, the biomolecule and the organic precursor are injected from a common container or from separate containers into the plasma afterglow.

In an implementation of the method, a water aerosol is injected together with the organic precursor and the biomolecule into the plasma afterglow during the APPD.

In an implementation of the method, argon or helium or nitrogen is used as plasma gas for the APPD.

In an implementation of the method, the codepositing of the organic precursor and the biomolecule forms a film on the surface.

The film is stable, for instance against aqueous dissolution, and has antifouling properties.

A second aspect of this disclosure provides a biosensor comprising: a surface on which a biomolecule is immobilized; wherein a film comprising the biomolecule and an organic precursor is formed on the surface; and wherein the organic precursor comprises a polymerizable group and an ethylene oxide functional group.

In an implementation of the biosensor, the film comprises a matrix formed by the ethylene oxide containing organic precursor and comprises the biomolecule dispersed in the matrix; and optionally the biomolecule is distributed in the film or on top of the film.

The biosensor of the first aspect enjoys the advantages explained above for the method of the first aspect. In particular, the layer on the surface is stable, retains the bioactivity of the biomolecule(s), and has antifouling properties.

### BRIEF DESCRIPTION OF THE DRAWINGS

The above described aspects and implementations are explained in the following description of embodiments with respect to the enclosed drawings:
- FIG. 1: schematically illustrates a method for functionalizing a surface of a biosensor with a biomolecule, according to this disclosure.
- FIG. 2: illustrates a device for performing APPD, the device being configured to codeposit the biomolecule and the organic precursor from a common container.
- FIG. 3: illustrates a device for performing APPD, the device being configured to codeposit the biomolecule and the organic precursor from separate containers.
- FIG. 4: shows (a) a biosensor with a functionalized surface according to this disclosure, and shows (b) examples of an organic precursor proposed in this disclosure.
- FIG. 5: shows images of functionalized surfaces comprising an organic precursor matrix and biomolecule.

### DETAILED DESCRIPTION OF EMBODIMENTS

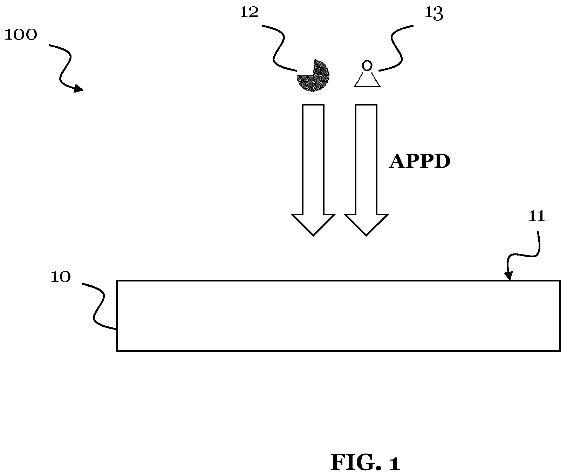

FIG. 1 illustrates a method 100 for functionalizing a surface 11 of a biosensor 10 with a biomolecule 12. The biomolecule 12 may be any one of an enzyme, an antibody, a nanobody, an aptamer, DNA, RNA, and a peptide. For example, the biomolecule 12 is glucose oxidase. The biosensor 10 may have a solid support structure, such as a metal, oxide, or carbon electrode formed by a substrate, a surface 11 of which may be applied with the biomolecule 12. During the APPD, the biosensor 10 may yet be in a pre-processed state, and the method 100 is used to functionalize a substrate surface, which will become the surface 11 of the biosensor 10 after processing the biosensor 10 is complete.

In an example, the biosensor 10 may be a glucose biosensor, where glucose oxidase enzymes may be functionalized on the electrode to detect blood sugar levels. In another example, the biosensor 10 may be an immunosensor, which uses antibodies on the sensor surface 11 to specifically bind target antigens for disease diagnostics.

The method 100 comprises codepositing the biomolecule 12 and an organic precursor 13 onto the surface 11 using APPD. The APPD is a deposition technique that uses a plasma at ambient pressure to deposit thin films on surfaces. APPD enables precise functionalization of materials by introducing reactive species that facilitate polymerization or grafting of biomolecules, making it useful for biosensor surface modification

The organic precursor 13 used in this disclosure comprises a polymerizable group and an ethylene oxide. The polymerizable group of the organic precursor 13 can comprise a vinyl, or an acrylate, or a methacrylate, or an epoxy, or a silane. For instance, the organic precursor 13 may be di(ethylene glycol) divinyl ether or may be poly(ethylene glycol) dimethacrylate. The organic precursor 13 may further comprise a spacer, for instance, an alkane, or an ether, or a ketone, ester, amide.

The organic precursor 13 used in this disclosure is purposefully selected, according to the following:
(1) The polymerizable group (e.g., vinyl, acrylate, methacrylate, epoxy, silane...) has the purpose to provide cross-linking.
(2) The ethylene oxide ((CH₂-CH₂-O)ₙ, n>=2) has the purpose to provide the antifouling properties.
(3) The optional spacer (alkane, ether, ketone ...) has the purpose to support the biomolecules functionality and bioactivity on the surface 11.

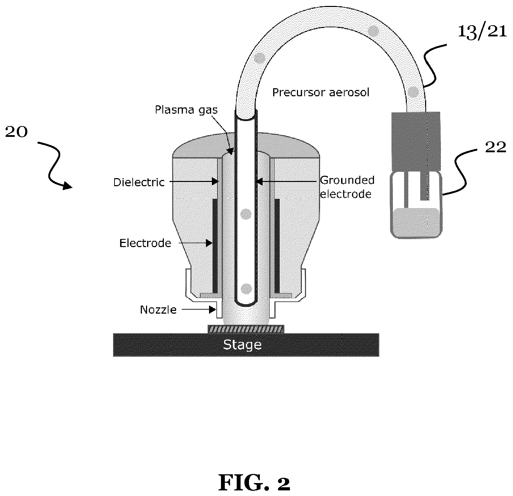

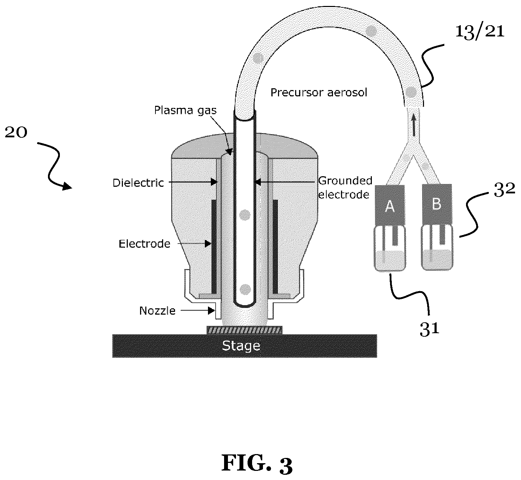

FIG. 2 and FIG. 3 illustrate exemplary devices 20 for codepositing the biomolecule 12 and the organic precursor 13 using APPD. The APPD devices 20 respectively generate a plasma in a chamber where a high-voltage electrode ionizes a flowing gas, while a grounded electrode stabilizes the discharge. The grounded electrode can but does not has to be a hollow electrode. A dielectric barrier prevents arcing, ensuring a uniform plasma that exits through a nozzle and directs the biomolecule 12 and organic precursor 13 onto the surface of a substrate or biosensor 10 placed on a stage. This device setup enables the precise codepositioning at atmospheric pressure without requiring a vacuum system.

The organic precursor 13 may be injected as a vapor or as an aerosol into the plasma afterglow, while the biomolecule 12 and a mediator may be injected as an aerosol. The biomolecule 12 can be either dissolved in the organic precursor 13, or can be injected as a separate aerosol. The organic precursor 13 and the biomolecule 12 (i.e., many molecules of the biomolecule 12) may then be copolymerized by the plasma afterglow, or may be immobilized in a different way, e.g., electrostatically or by steric embedding. This provides stability to the biomolecule 12, preventing it from escaping from the film on the surface 11. Alternatively, the biomolecules are trapped in the film by the polymerized organic precursor matrix.

The difference between the setups of the device 20 shown FIG. 2 and FIG. 3, respectively, is that with the device 20 of FIG. 2 the biomolecule 12 and the organic precursor 13 are injected from a common container 22, while with the device 20 of FIG. 3 the biomolecule 12 and the organic precursor 13 are injected from two separate containers 31 and 32 into the plasma afterglow.

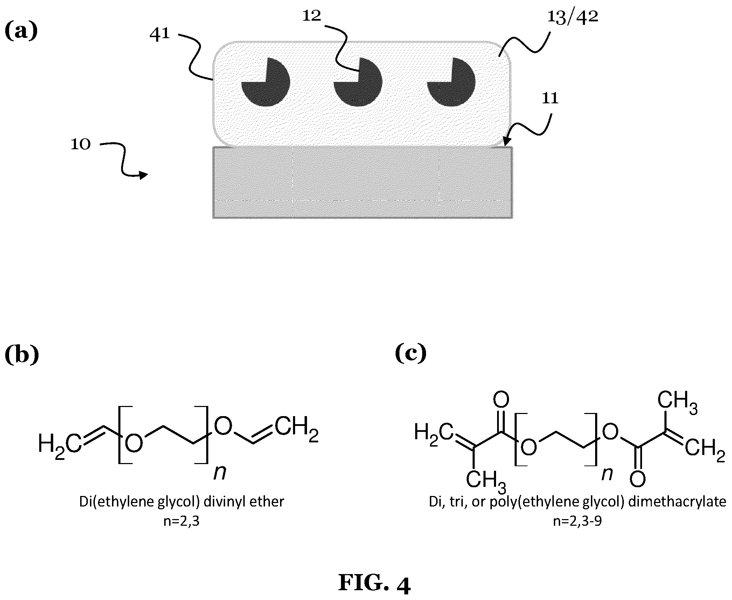

As shown in FIG. 4(a), the codeposition of the biomolecule 12 and the organic precursor 13 using APPD leads to a surface 11, on which the biomolecule 12 is functionalized. A film 41 comprising the biomolecule 12 and the organic precursor 13 may be formed on the surface 11. The film 41 may comprise a matrix 42 formed by (derived from) the ethylene oxide containing organic precursor 13, and comprises the (molecules of the) biomolecule 12 dispersed in the matrix 42. Thereby, the molecules of the biomolecule 12 may also be distributed on the surface of the matrix 42.

Codeposition of di(ethylene glycol) divinyl ether, which is shown in FIG. 4(b), as the organic precursor 13 and glucose oxidase as the biomolecule 12 were codeposited in an example. Another example of the organic precursor 13 is shown in FIG. 4(c).

The organic precursor 13 and the biomolecule 12 were injected as separate aerosols into the plasma afterglow. Argon was used as a plasma gas, as it limits heating of the surface, and the air content in the environment was controlled during deposition of the film 41. The resulting cross-linked polymer film 41 (schematically illustrated in FIG. 4(a)) is stable in an aqueous environment, has antifouling properties, and contains glucose oxidase with retention of its enzymatic activity, as the film 41 is functional for the electrochemical detection of glucose. The obtained film 41 is ideally suited for bio sensing, and meets the following three criteria:
The stability of the film 41 in the aqueous environment is achieved thanks to the polymerizable groups of the organic precursor 13, which are activated by the plasma of the APPD. The substrate surface providing the surface 11 can also be activated by the plasma, improving the adhesion.

The film 41 is further antifouling thanks to the ethylene oxide contained in the organic precursor 13. The organic precursor 13 particularly prevents fouling by proteins or cells. Organic precursors 13 with high ethylene oxide content are beneficial, which is made possible by injecting the organic precursor 13 as an aerosol.

The biomolecules 12 in the film 41 are still active, because the biomolecule 12 only contacts the plasma afterglow, reducing the fragmentation and/or denaturation of the biomolecule 12. A plasma close to room temperature and with a low energy can be used for cross-linking the film thanks to the polymerizable groups.

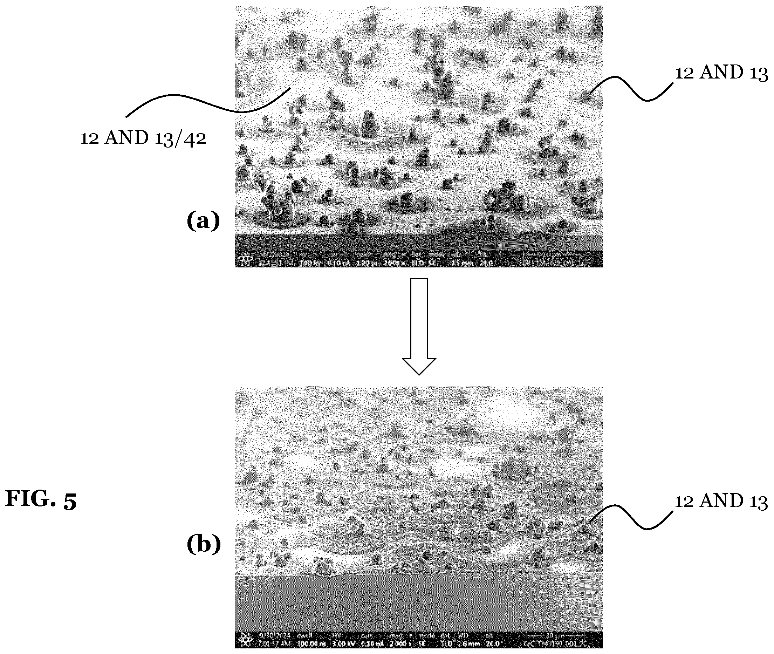

FIG. 5 shows images of a functionalized surface comprising a matrix of an organic precursor 13 and a biomolecule 12. Thereby, FIG. 5(a) is an image of an organic precursor matrix obtained without the use of a water aerosol, and FIG. 5(b) is an image of an organic precursor matrix 13 and a biomolecule 12, with addition of a water aerosol. Both images are the result of codepositing with APPD from a single container 22 (as shown in FIG. 2). The biomolecule 12 and the organic precursor 13 are mixed in both cases. Only in FIG. 5(b) they are more homogeneously distributed.

In sum, this disclosure provides a solution for functionalizing a surface with a biomolecule, wherein the use of APPD enables a one-step process, and the selection of the organic precursor 13 leads to a stable layer with strong cross-linking and antifouling properties.

In the claims as well as in the description of this disclosure, the word "comprising" does not exclude other elements or steps and the indefinite article "a" or "an" does not exclude a plurality. A single element may fulfill the functions of several entities or items recited in the claims. The mere fact that certain measures are recited in the mutual different dependent claims does not indicate that a combination of these measures cannot be used in an advantageous implementation.

## Claims

1. A method (100) for functionalizing a surface (11) of a biosensor (10) with a biomolecule (12), the method (100) comprising:
codepositing the biomolecule (12) and an organic precursor (13) onto the surface (11) using atmospheric pressure plasma deposition, APPD (20);
wherein the organic precursor (13) comprises a polymerizable group and an ethylene oxide functional group.

2. The method (100) of claim 1, wherein
the polymerizable group of the organic precursor (13) comprises a vinyl, or an acrylate, or a methacrylate, or an epoxy, or a silane.

3. The method (100) of claim 1 or 2, wherein
the organic precursor (13) further comprises a spacer, the spacer comprising an alkane, or an ether, or a ketone, or an ester, or an amide.

4. The method (100) of one of the claims 1 to 3, wherein
the biomolecule (12) is one of an enzyme, an antibody, a nanobody, an aptamer, DNA, RNA, and a peptide.

5. The method (100) of one of the claims 1 to 4, wherein
the organic precursor (13) is di(ethylene glycol) divinyl ether or poly(ethylene glycol) dimethacrylate, and/or the biomolecule (12) is glucose oxidase.

6. The method (100) of one of the claims 1 to 5, wherein
the biomolecule (12) and the organic precursor (13) are codeposited assisted by a plasma afterglow during the APPD (20) or are codeposited and then treated with the plasma afterglow.

7. The method (100) of claim 6, wherein
during the APPD (20), the organic precursor (13) is injected as vapor or an aerosol (21) into the plasma afterglow, and the biomolecule (12) is injected as an aerosol into the plasma afterglow.

8. The method (100) of claim 6, wherein
the biomolecule (12) and the organic precursor (13) are dissolved in water, and during the APPD (20) the water comprising the dissolved organic precursor (13) and biomolecule (12) is injected into the plasma afterglow.

9. The method (100) of one of the claims 6 to 8, wherein
the biomolecule (12) and the organic precursor (13) are injected from a common container (22) or from separate containers (31, 32) into the plasma afterglow.

10. The method (100) of one of the claims 6 to 9, wherein
a water aerosol is injected together with the organic precursor (13) and the biomolecule (12) into the plasma afterglow during the APPD (20).

11. The method (100) of one of the claims 1 to 10, wherein
argon or helium or nitrogen is used as plasma gas for the APPD (20).

12. The method (100) of one of the claims 1 to 11, wherein
the codepositing of the organic precursor (13) and the biomolecule (12) forms a film (41) on the surface (11).

13. A biosensor (10) comprising:
a surface (11) on which a biomolecule (12) is immobilized;
wherein a film (41) comprising the biomolecule (12) and an organic precursor (13) is formed on the surface (11); and
wherein the organic precursor (13) comprises a polymerizable group and an ethylene oxide functional group.

14. The biosensor (10) of claim 13, wherein
the film (41) comprises a matrix (42) formed by the ethylene oxide containing organic precursor (13) and comprises the biomolecule (12) dispersed in the matrix (42); and
optionally the biomolecule (12) is distributed in the film (41) or on top of the film (41).
